# EUROPEAN PATENT SPECIFICATION

(11) **EP 4 536 297 B1**
(45) Date of publication and mention of the grant of the patent: **27.05.2026**
(21) Application number: 23730053.8
(22) Date of filing: 30.05.2023
(51) Int. Cl.: A61L 2/18

(54) **DEVICE FOR DISINFECTING AND CLEANING A CATHETER, AND A METHOD THEREOF**
VORRICHTUNG ZUR DESINFEKTION UND REINIGUNG EINES KATHETERS UND VERFAHREN DAFÜR
DISPOSITIF DE DÉSINFECTION ET DE NETTOYAGE D'UN CATHÉTER, ET PROCÉDÉ ASSOCIÉ

(30) Priority: 08.06.2022 IN 202241032764
(43) Date of publication of application: 16.04.2025
(73) Proprietor: Gambro Lundia AB, 226 43 Lund (SE)
(72) Inventor: APORVA, Raj M R, Gopala Karnataka, Shivamogga 577205 (IN)
(74) Representative: Sweden SHS IP Office
(86) International application number: PCT/EP2023/064371
(87) International publication number: WO 2023/237378

(56) References cited:
- WO-A1-2015/148427
- WO-A2-2010/062589
- US-A1- 2004 187 893
- US-A1- 2011 023 885

## Description

### FIELD OF INVENTION

The present invention relates to a device for disinfecting and cleaning a catheter.

### BACKGROUND OF THE INVENTION

The subject matter discussed in the background section should not be assumed to be prior art merely as a result of its mention in the background section. Similarly, a problem mentioned in the background section are associated with the subject matter of the background section should not be assumed to have been previously recognized in the prior art. The subject matter in the background section merely represents different approaches, which in and of themselves may also correspond to implementations of the claimed technology.

Catheters are thin and flexible tubes with varying levels of stiffness depending on their application. Catheters are inserted in a body cavity, duct, vessel, skin, or adipose tissues, to treat diseases or to perform a surgical procedure. By modifying the structure and material of the catheters, it is possible to tailor catheters for cardiovascular, urological, gastrointestinal, neurovascular, and ophthalmic applications. Catheters allow drainage, administration of fluids or gases, or access of a body cavity.

Central Venous Catheters (CVCs), Peripherally Inserted Central Catheters (PICCs) and Peritoneal dialysis (PD) Catheters are indispensible devices used vastly in medical practice, particularly among critically ill patients, cancer patients, and those receiving intravenous feeding. These catheters allow for the rapid infusion of concentrated medications, fluids, or blood products that otherwise cannot be administered into peripherally extreme veins via standard intravenous probes.

Although these catheters (CVCs, PICCs and PD catheters) provide necessary vascular access, they also put the patients at risk of significant infection, which can be life-threatening. Most Catheter-Related Bloodstream Infections (CRBI) are associated with endoluminal contamination and subsequent colonization of the catheters during their long-term usage. For instance, a PD catheter is prone to develop biofilm, fibrin sheath, bacterial build-up, and scarring formation at inner diameter because of continuous presence of fluid. A catheter affected with such factors causes thrombosis and peritonitis in patients. Generally, a catheter becomes contaminated when micro-organisms settle over its external surface by coming in contact with skin of a patient or an operator, tracheostomy secretions, wounds, and the like. A contaminated catheter can lead to Central Line-Associated Bloodstream Infection (CLABSI), a serious infection that occurs when germs, usually bacteria or viruses, enter the bloodstream of a patient through a contaminated catheter. Such infections also require unplanned removal of contaminated catheters.

Efforts have been made in the past to disinfect catheters using stringent infection control practices. Some of these stringent infection control practices include usage of fluids to disinfect a catheter each time after its usage. A typical example is Citra-lock^{™} which is a solution prefilled into the catheter after every procedure, as an antibacterial, anticoagulant, and anti-biofilm fluid. However, such techniques are not effective for sterilization and disinfection of harmful micro-organisms settled inside and biofilms deposited inside the catheter.

Currently available catheters are designed primarily to ensure a tight connection with an intravenous tubing, but are not designed specifically to prevent contamination, such as endoluminal catheter microbial colonization.

WO 2010/062589 A2 discloses a system for delivering an antimicrobial agent into the lumen of a trans-dermal catheter. In an embodiment, the system comprises an elongate member configured for insertion into a lumen of a catheter; an expandable of the elongate member, said expandable portion configured to increase in diameter upon exposure to an aqueous fluid; and an antimicrobial composition positioned to be delivered into the catheter.

US 2011/023885 A1 discloses systems, devices, and methods for the cleaning of an endotracheal tube while a patient is supported by a ventilator connected to the endotracheal tube for the purpose of increasing the available space for airflow or to prevent the build up of materials that may constrict airflow or be a potential nidus for infection.

US 2004/187893 A1 presents a cleaning element comprising a brush having a plurality of bristles coated with a hydrophilic polymer.

WO 2018/148427 provides a composition for monitoring the efficacy of a decontamination process, the composition comprising a cellulose polymer and a predetermined quantity of an adenine nucleotide.

Therefore, there exists a need for techniques and devices that are effective in removal of microbial contamination of a catheter. The present invention satisfies this need and envisages to provide related advantages as well.

### OBJECTS OF THE INVENTION

An objective of the present invention is to provide a device for disinfecting and cleaning a catheter, and a method thereof.

Another objective is to provide a device capable of preventing bloodstream infections in patients, and a method thereof.

Another objective is to provide a device and a method capable of disinfecting inner surface of a catheter and removing microbial contamination.

Still another objective is to provide a device to preventing formation of fibrin sheath inside the catheter, and a method thereof.

### SUMMARY OF THE INVENTION

The present invention is defined in the appended claims. An aspect of the present invention relates to a device for disinfecting and cleaning inner surface of a catheter to remove microbial contamination and prevent formation of fibrin sheath in the catheter. The device includes a stem, and an expandable member fixed at a front portion of the stem, the expandable member wetted with a disinfectant. The device also includes a sheath assembly housing the stem and the expandable member. The sheath assembly includes a front end that is closed, and a plurality of slots present near the front end.

The plurality of slots allow for outward expansion of the expandable member when the expandable member is pushed towards the front end by the stem, and allow for inward movement of the expandable member when the expandable member is pushed away from the front end by the stem. When the sheath assembly is inserted within the catheter and the expandable member is brought outward by pushing through the stem, the expandable member wetted with the disinfectant comes in contact with inner surface of the catheter, thereby disinfecting the catheter.

In one aspect, the expandable member is selected from a group consisting of a sponge, a foam, and cotton.

In one aspect, the front end is a sheath cover removably secured with a body of the sheath assembly.

In one aspect, the stem is rotated while being pushed towards the front end to allow outward expansion of the expandable member through the plurality of slots, to completely clean the inner surface of the catheter by removing any biofilm and killing harmful micro-organisms.

In one aspect, the front end is coated with an anticoagulant.

Another aspect of the present invention relates to a method for disinfecting inner surface of a catheter. The method includes inserting a device for disinfecting the catheter into the catheter, pushing the expandable member towards the front end by operating the stem to allow outward expansion of the expandable member through the plurality of slots when the expandable member reaches the front end, and pulling the device out of the catheter when the expandable member is present in the outwardly expanded form, thereby disinfecting the catheter.

In one aspect, the method includes rotating the device while pulling the device out of the catheter. Inserting the device into the catheter includes inserting the sheath assembly into any of an arterial line or a venous line of the catheter. The method also includes flushing the arterial line and the venous line of the catheter with a saline solution before inserting the device within, and removing residual saline solution from the arterial line and the venous line after pulling the device out of the catheter.

### BRIEF DESCRIPTION OF THE DRAWINGS

The accompanying drawings constitute a part of the description and are used to provide a further understanding of the present invention. The drawings illustrate exemplary embodiments of the present disclosure and, together with the description, serve to explain the principles of the present disclosure.
Figs. 1A and 1B illustrate sectional side views of a device for disinfecting a catheter in inactivated and activated states respectively, in accordance with an embodiment of the present invention.
Figs. 2A and 2B illustrate perspective side views of a device for disinfecting a catheter in inactivated and activated states respectively, in accordance with an embodiment of the present invention.
Figs. 3A and 3B illustrate side views of a device for disinfecting a catheter being inserted into an arterial line and a venous line of the catheter, respectively, in accordance with an embodiment of the present invention.
Fig. 4 illustrates a perspective side view of a device for disinfecting a catheter fully inserted into an arterial line of the catheter, in accordance with an embodiment of the present invention.
Fig. 5 illustrates a device used for disinfecting a catheter disposed in a sterile packaging, in accordance with an embodiment of the present invention.
Fig. 6 illustrates a flowchart showing a method for disinfecting inner surface of a catheter, in accordance with an embodiment of the present invention.

### DETAILED DESCRIPTION OF THE INVENTION

The detailed description set forth below in connection with the appended drawings is intended as a description of various embodiments of the present invention and is not intended to represent the only embodiments in which the present invention may be practiced. Each embodiment described in this disclosure is provided merely as an example or illustration of the present invention, and should not necessarily be construed as preferred or advantageous over other embodiments. The detailed description includes specific details for the purpose of providing a thorough understanding of the present invention. However, it will be apparent to those skilled in the art that the present invention may be practiced without these specific details.

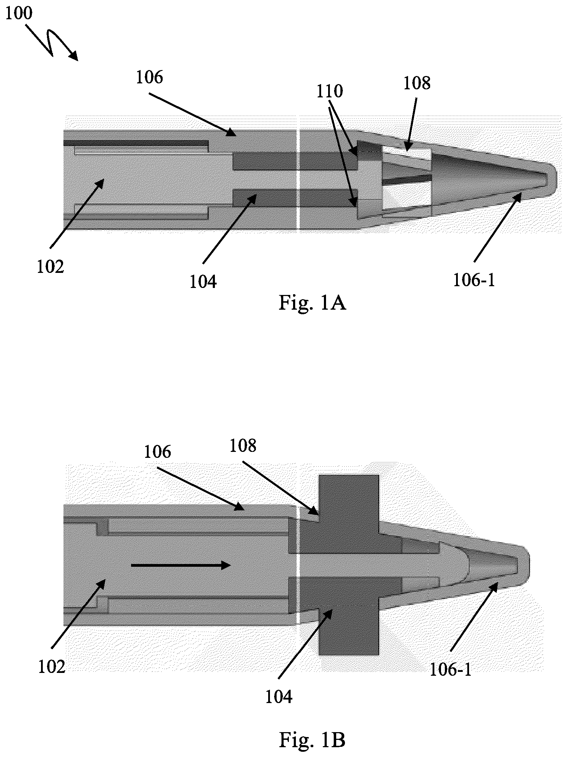

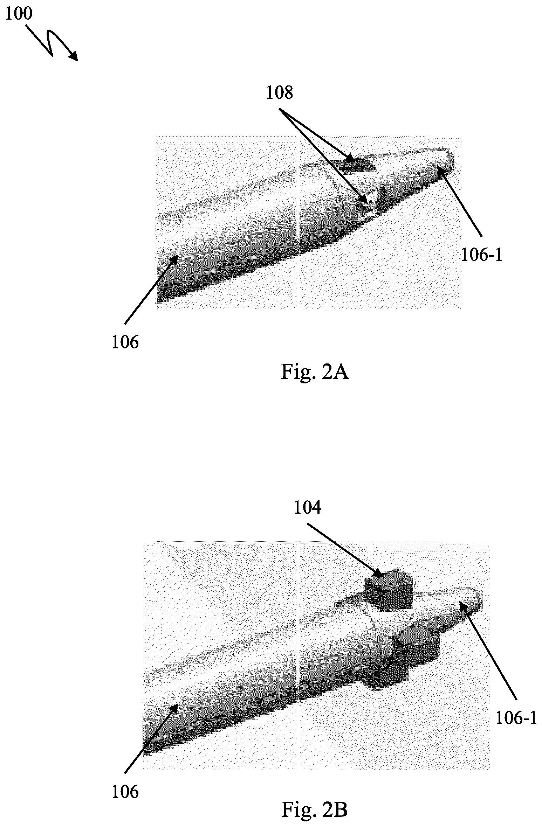

The present invention relates to a device for disinfecting and cleaning inner surface of a catheter to remove microbial contamination and prevent formation of fibrin sheath in the catheter, and a method thereof. Figs. 1A and 1B illustrate sectional side views of a device **100** for disinfecting a catheter. Figs. 2A and 2B illustrate perspective side views of the device **100** in inactivated and activated states respectively. The device **100** includes a stem **102** and an expandable member **104** fixed at a front end **102-1** of the stem **102.** The expandable member **104** is wetted with a disinfectant, such as Povidone-iodine solution, saline solution, and citric acid. In certain implementations, citric acid of 0.5-1% concentration may be used as the disinfectant. The expandable member **104** may be selected as a polyurethane flexible foam having excellent absorption properties, when dipped with the disinfectant. Another criteria for selecting the expandable member **104** may be the flexibility/elasticity of the expandable member **104.** The expandable member **104** is selected from a group consisting of a sponge, a foam, cotton, silicone rubber, polyurethane, or plastic mesh.

The device **100** also includes a sheath assembly **106** housing the stem **102** and the expandable member **104.** The sheath assembly **106** includes a front end **106-1** that is closed, and a plurality of slots **108** present near the front end **106-1.** The front end **106-1** is a sheath cover removably secured with a body of the sheath assembly **106.** The front end **106-1** is coated with a suitable antithrombotic and antimicrobial coating to prevent thrombosis and catheter-related infection. The antithrombotic and antimicrobial coating may be prepared using Heparin, silver, chlorhexidine, rifampicine, or minocycline. In one implementation, the front end **106-1** may be coated with an anticoagulant, such as Heparin.

The plurality of slots **108** allow for outward expansion of the expandable member **104** when the expandable member **104** is pushed towards the front end **106-1** by operating the stem **102.** Further, after a disinfection operation is completed, the expandable member **104** may be pulled away from the front end **106-1** and brought inward to come in a compressed state for safe disposal, by operating the stem **102.**

In the inactivated state as shown in Fig. 2A, the expandable member **104** is present in a non-expanded/compressed position i.e. the expandable member **104** is not expanded out of the slots **108** of the sheath assembly **106.** When the stem **102** is moved towards the front end **106-1** of the sheath assembly **106** (indicated by an arrow in Fig. 2B), the expandable member **104** wetted with the disinfectant expands by coming out of the slots **108** of the sheath assembly **106** and contacts the inner surface of the catheter, in the activated state. The device **100** may then be pulled away from the front end **106-1** of the sheath assembly **106** to enable disinfecting and cleaning of the inner surface of the catheter. Also, the expandable member **104** may be rotated, clockwise and/or counter clockwise, when the expandable member **104** is present in the activated state, for complete cleaning of the inner surface of the catheter, and remove any biofilm. Such manner of cleaning would prevent formation of fibrin sheath inside the catheter. The stem **102** may be removably provided such that the stem **102** is pushed into the sheath assembly **106** only during the procedure of disinfecting the catheter.

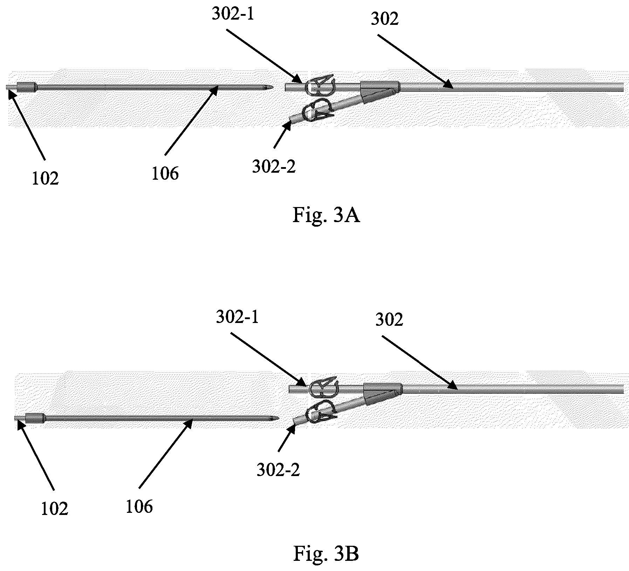

As shown in Figs. 3A and 3B, the device **100** may be fully inserted into an arterial line **302-1** or a venous line **302-2** of a catheter **302,** such as a high-flow dolphin catheter, to disinfect and clean the arterial line **302-1** or the venous line **302-2.** The stem **102** may be pushed towards the front end **106-1** to enable outward expansion of the expandable member **104** wetted with the disinfectant, and contact the inner surface of the arterial line **302-1.** The device **100** may then be pulled away from the front end **106-1** to enable disinfecting and cleaning of the inner surface of the catheter **302.**

While the expandable member **104** is present in the activated state, the expandable member **104** may be rotated to completely clean and sanitize the inner surface of the arterial line **302-1** and prevent formation of fibrin sheath inside the arterial line **302-1.** Further, as shown in Fig. 3B, the sheath assembly **106** may be inserted into the venous line **302-2** of the catheter **302,** to disinfect and clean the venous line **302-2** in a similar manner. While pulling the device **100** away from the front end **106-1,** the device **100** may be rotated to completely clean the inner surface of the catheter **302.** This would remove any biofilm and prevent formation of fibrin sheath inside the catheter **302.**

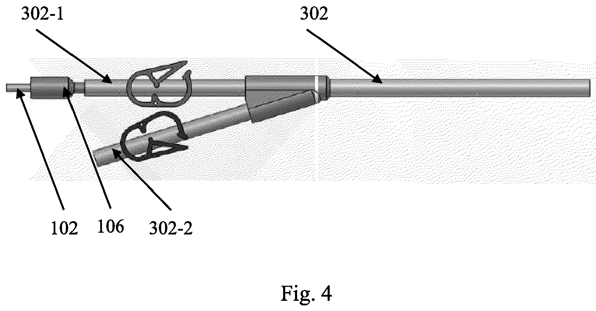

Fig. 4 illustrates a perspective view of the device **100** fully inserted into the arterial line **302-1** of the catheter **302.** Length of the sheath assembly **106** of the device **100** may be larger than the catheter **302** in which the device **100** may need to be inserted. The sheath assembly **106** accommodating the stem **102** and the expandable member **104** may be inserted into any of the arterial line **302-1** or the venous line **302-2** of the catheter **302,** such that the front end **106-1** of the sheath assembly **106** is in proximity of a tip of the catheter **302,** to allow disinfection and cleaning of entire length of the catheter **302.**

When the sheath assembly **106** accommodating the stem **102** and the expandable member **104** is inserted into the catheter **302,** a rear portion of the stem **102** may extend beyond a rear portion of the sheath assembly **106,** to allow a user to move the stem **102** towards the front end **106-1** of the sheath assembly **106** and gradually pulling the device **100** out of the catheter **302** for completing the disinfection procedure. It must be noted that the expandable member **104** cannot be pulled back into the sheath assembly **106** when the disinfection procedure is being carried out. Referring back to Fig. 1A, a front inner surface **110** (adjacent to the slots **108)** of the sheath assembly **106** may act as a barrier for movement of the expandable member **104** within the sheath assembly **106,** which may be caused due to accidental pulling back of the expandable member **104** during the disinfection procedure.

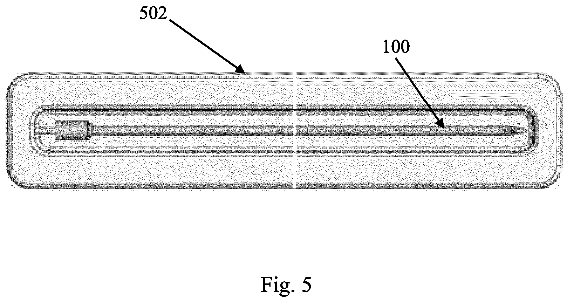

Once the disinfection procedure is completed, the expandable member **104** contaminated with biofilm or microbes may be pulled back with force into the sheath assembly **106,** through the slots **108.** Pulling of the expandable member **104** into the sheath assembly **106** would prevent the expandable member **104** from coming in contact with objects or surfaces outside the catheter **302,** thereby preventing spreading of the contamination. Bringing the expandable member **104** back into the sheath assembly **106** after the disinfection procedure would help in safe disposal of the device **100,** as the device **100** may be developed for single time use in certain implementations. Additionally, the device **100** may be enclosed in a sterile packaging **502** before disposal, as illustrated in Fig. 5. The sterile packaging **502** may be made of plastic, glass, or any other suitable material. Once closed, the sterile packaging **502** may become air locked and may not allow leakage of contaminants to the outside.

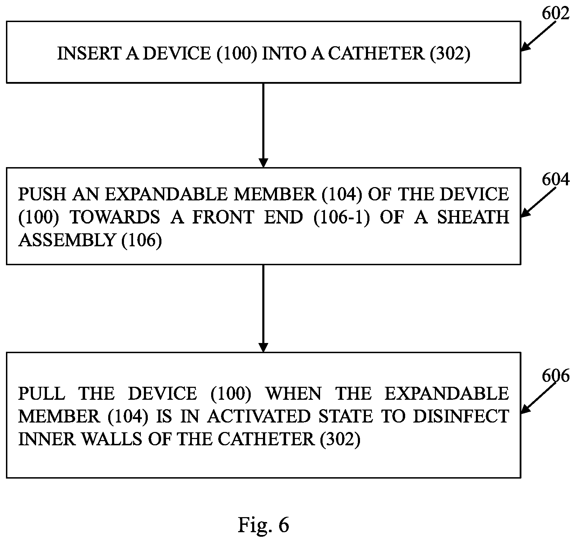

Fig. 6 illustrates a flowchart of a method for disinfecting the catheter, in accordance with an embodiment of the present invention. The method includes, at step **602,** inserting a device **100** for disinfecting a catheter **302** into the catheter **302.** The device includes a stem **102,** an expandable member **104** fixed at a front end **102-1** of the stem **102,** and a sheath assembly **106** housing the stem **102** and the expandable member **104.** The expandable member **104** is wetted with a disinfectant. When the device **100** is inserted into the catheter **302,** the sheath assembly **106** may be inserted into any of an arterial line **302-1** or a venous line **302-2** of the catheter **302** (such as a dolphin catheter).

As a precautionary measure, before inserting the sheath assembly **106** into the catheter **302,** the catheter **302** may be flushed with a saline solution to prevent contact of residual blood which may be present in the catheter **302** with the device **100.**

The method further includes, at step **604,** pushing the expandable member **104** towards the front end **106-1** of the sheath assembly **106** by operating the stem **102,** to allow outward expansion of the expandable member **104.** A plurality of slots **108** are present near the front end **106-1** of the sheath assembly **106,** which allow for outward expansion of the expandable member **104** through the slots **108** when the expandable member **104** is pushed towards the front end **106-1** by the stem **102** and reaches the front end **106-1.**

The method includes, at step **606,** pulling the device **100** out of the catheter **302** when the expandable member **104** is present in an activated state, i.e., in an outwardly expanded form. The device **100** is pulled away from the front end **106-1** of the sheath assembly **106** to enable disinfecting and cleaning of inner surface of the catheter **302,** while the expandable member **104** is present in the activated state, so that the inner surface of the catheter **302** is completely disinfected and cleaned, and any residual biofilm present in the catheter **302** is removed. Also, while the device **100** is being pulled away from the front end **106-1,** the device **100** may be rotated, either clockwise or counter clockwise, to complete clean the inner surface of the catheter **302** and prevent formation of fibrin sheath inside the catheter **302.**

After the device **100** is pulled out of the catheter **302,** any residual saline solution may be removed from the catheter **302** to remove traces of any residual biofilms. Thereafter, new injection line caps may be used to close inlet ports of the catheter **302.**

Thus, the present invention provides a device for disinfecting and cleaning catheters, and a method thereof. The device is capable of capable of efficiently disinfecting inner surface of the catheters and removing microbial contamination of the catheters, to prevent bloodstream infections in patients. The device and the method described in present invention also prevent formation of fibrin sheath and traces of biofilm in the catheters. The device or one or more of its components may be replaced and/or disposed after cleaning and disinfecting a catheter, to ensure safety of the disinfection procedure. The device prevents usage of heparin lock solutions which typically imparts more weight on a catheter and increase pressure thereto. The device is easy to use and cost effective, while reducing operational time for disinfection of a catheter.

In the above detailed description, reference is made to the accompanying drawings that form a part thereof, and illustrate the best mode presently contemplated for carrying out the invention. However, such description should not be considered as any limitation of scope of the present unit. The structure thus conceived in the present description is susceptible of numerous modifications and variations, all the details may furthermore be replaced with elements having technical equivalence.

## Claims

1. A device (100) for disinfecting a catheter (302), comprising:
a stem (102);
an expandable member (104) fixed at a front portion of the stem (102), wherein the expandable member (104) is wetted with a disinfectant; and
a sheath assembly (106) housing the stem (102) and the expandable member (104), wherein the sheath assembly (106) includes:
a front end (106-1) that is closed; and
a plurality of slots (108) present near the front end (106-1), wherein the plurality of slots (108) allow for outward expansion of the expandable member (104) when the expandable member (104) is pushed towards the front end (106-1) by the stem (102),
wherein the sheath assembly (106) is configured to be inserted within the catheter (302) and the expandable member (104) is configured to be brought outward by pushing through the stem (102), the expandable member (104) wetted with the disinfectant is configured to come in contact with an inner surface of the catheter (302), thereby disinfecting the catheter (302).

2. The device (100) as claimed in claim 1, wherein the expandable member (104) is selected from a group consisting of sponge, foam, and cotton.

3. The device (100) as claimed in claim 1, wherein the front end (106-1) is a sheath cover removably secured with the sheath assembly (106).

4. The device (100) as claimed in claim 1, wherein the front end (106-1) is coated with an anticoagulant.

5. The device (100) as claimed in claim 1, wherein the sheath assembly (106) is configured to be inserted into any of an arterial line (302-1) or a venous line (302-2) of the catheter (302) for disinfection.

6. A non-therapeutical method for disinfecting a catheter (302), the method comprising:
inserting a device (100) for disinfecting the catheter (302) into the catheter (302), the device (100) comprising:
an expandable member (104) fixed at a front portion of a stem (102) and wetted with a disinfectant; and
a sheath assembly (106) housing the stem (102) and the expandable member (104), wherein the sheath assembly (106) includes a front end (106-1) that is closed and a plurality of slots (108) that are present near the front end (106-1);
pushing the expandable member (104) towards the front end (106-1) by operating the stem (102), to allow outward expansion of the expandable member (104) through the plurality of slots (108) when the expandable member (104) reaches the front end (106-1), wherein the expandable member (104) wetted with the disinfectant and present in an outwardly expanded form makes contact with an inner surface of the catheter (302); and
pulling the device (100) out of the catheter (302) when the expandable member (104) is present in the outwardly expanded form, thereby disinfecting the catheter (302).

7. The non-therapeutical method as claimed in claim 6, comprising rotating the device (100) while pulling the device (100) out of the catheter (302).

8. The non-therapeutical method (500) as claimed in claim 6, wherein inserting the device (100) into the catheter (302) comprises inserting the sheath assembly (106) into any of an arterial line (302-1) or a venous line (302-2) of the catheter (302).

9. The non-therapeutical method (500) as claimed in claim 8, comprising:
flushing the arterial line (302-1) and the venous line (302-2) of the catheter (302) with a saline solution before inserting the device (100) within; and
removing residual saline solution from the arterial line (302-1) and the venous line (302-2) after pulling the device (100) out of the catheter (302).

10. The non-therapeutical method (500) as claimed in claim 6, wherein the expandable member (104) is pulled back within the sheath assembly (106) after completion of a disinfection procedure, for safe disposal of the device (100).

## Patentansprüche

1. Vorrichtung (100) zum Desinfizieren eines Katheters (302), umfassend:
einen Schaft (102),
ein expandierbares Glied (104), das an einem vorderen Abschnitt des Schafts (102) befestigt ist, wobei das expandierbare Glied (104) mit einem Desinfektionsmittel benetzt ist, und
eine Hülsenbaugruppe (106), in der der Schaft (102) und das expandierbare Glied (104) untergebracht sind, wobei die Hülsenbaugruppe (106) Folgendes aufweist:
ein vorderes Ende (106-1), das geschlossen ist, und
eine Vielzahl von Schlitzen (108), die in der Nähe des vorderen Endes (106-1) vorliegen, wobei die Vielzahl von Schlitzen (108) eine nach außen gerichtete Expansion des expandierbaren Glieds (104) gestatten, wenn das expandierbare Glied (104) von dem Schaft (102) zu dem vorderen Ende (106-1) hin geschoben wird,
wobei die Hülsenbaugruppe (106) dazu ausgestaltet ist, in den Katheter (302) eingeführt zu werden, und das expandierbare Glied (104) dazu ausgestaltet ist, durch Schieben durch den Schaft (102) nach außen gebracht zu werden, wobei das mit dem Desinfektionsmittel benetzte expandierbare Glied (104) dazu ausgestaltet ist, mit einer Innenfläche des Katheters (302) in Kontakt zu kommen, wodurch der Katheter (302) desinfiziert wird.

2. Vorrichtung (100) nach Anspruch 1, wobei das expandierbare Glied (104) aus einer Gruppe ausgewählt ist, die aus Schwamm, Schaumstoff und Watte besteht.

3. Vorrichtung (100) nach Anspruch 1, wobei das vordere Ende (106-1) eine Hülsenabdeckung ist, die entfernbar an der Hülsenbaugruppe (106) befestigt ist.

4. Vorrichtung (100) nach Anspruch 1, wobei das vordere Ende (106-1) mit einem Antikoagulans beschichtet ist.

5. Vorrichtung (100) nach Anspruch 1, wobei die Hülsenbaugruppe (106) dazu ausgestaltet ist, zur Desinfizierung in ein arterielles Lumen (302-1) oder ein venöses Lumen (302-2) des Katheters (302) eingeführt zu werden.

6. Nicht-therapeutisches Verfahren zum Desinfizieren eines Katheters (302), wobei das Verfahren Folgendes umfasst:
Einführen einer Vorrichtung (100) zum Desinfizieren des Katheters (302) in den Katheter (302), wobei die Vorrichtung (100) Folgendes umfasst:
ein expandierbares Glied (104), das an einem vorderen Abschnitt eines Schafts (102) befestigt und mit einem Desinfektionsmittel benetzt ist, und
eine Hülsenbaugruppe (106), in der der Schaft (102) und das expandierbare Glied (104) untergebracht sind, wobei die Hülsenbaugruppe (106) ein vorderes Ende (106-1), das geschlossen ist, und eine Vielzahl von Schlitzen (108) aufweist, die nahe dem vorderen Ende (106-1) vorliegen, durch Betätigen des Schafts (102) Schieben des expandierbaren Glieds (104) auf das vordere Ende (106-1) zu, um eine nach außen gerichtete Expansion des expandierbaren Glieds (104) durch die Vielzahl von Schlitzen (108) zu gestatten, wenn das expandierbare Glied (104) das vordere Ende (106-1) erreicht, wobei das mit dem Desinfektionsmittel benetzte und in einer nach außen expandierten Form vorliegende expandierbare Glied (104), Kontakt mit einer Innenfläche des Katheters (302) herstellt, und
Ziehen der Vorrichtung (100) aus dem Katheter (302), wenn das expandierbare Glied (104) in der nach außen expandierten Form vorliegt, wodurch der Katheter (302) desinfiziert wird.

7. Nicht-therapeutisches Verfahren nach Anspruch 6, umfassend das Drehen der Vorrichtung (100), während die Vorrichtung (100) aus dem Katheter (302) gezogen wird.

8. Nicht-therapeutisches Verfahren (500) nach Anspruch 6, wobei das Einführen der Vorrichtung (100) in den Katheter (302) das Einführen der Hülsenbaugruppe (106) in ein arterielles Lumen (302-1) oder ein venöses Lumen (302-2) des Katheters (302) umfasst.

9. Nicht-therapeutisches Verfahren (500) nach Anspruch 8, umfassend:
Spülen des arteriellen Lumens (302-1) und des venösen Lumens (302-2) des Katheters (302) mit einer Kochsalzlösung, bevor die Vorrichtung (100) in diese eingeführt wird, und
Entfernen von restlicher Kochsalzlösung aus dem arteriellen Lumen (302-1) und dem venösen Lumen (302-2) nach Herausziehen der Vorrichtung (100) aus dem Katheter (302) .

10. Nicht-therapeutisches Verfahren (500) nach Anspruch 6, wobei das expandierbare Glied (104) nach Abschluss eines Desinfektionsvorgangs zur sicheren Entsorgung der Vorrichtung (100) in der Hülsenbaugruppe (106) zurückgezogen wird.

## Revendications

1. Dispositif (100) pour la désinfection d'un cathéter (302), comprenant :
une tige (102) ;
un élément déployable (104) fixé au niveau d'une partie avant de la tige (102), l'élément déployable (104) étant humidifié avec un désinfectant ; et
un ensemble gaine (106) logeant la tige (102) et l'élément déployable (104), l'ensemble gaine (106) comportant :
une extrémité avant (106-1) qui est fermée ; et
une pluralité de fentes (108) présentes près de l'extrémité avant (106-1), la pluralité de fentes (108) permettant le déploiement vers l'extérieur de l'élément déployable (104) lorsque l'élément déployable (104) est poussé vers l'extrémité avant (106-1) par la tige (102), dans lequel l'ensemble gaine (106) est configuré pour être inséré dans le cathéter (302) et l'élément déployable (104) est configuré pour être poussé vers l'extérieur à travers la tige (102), l'élément déployable (104) humidifié avec le désinfectant est configuré pour venir en contact avec une surface interne du cathéter (302), désinfectant de ce fait le cathéter (302).

2. Dispositif (100) selon la revendication 1, dans lequel l'élément déployable (104) est choisi dans un groupe constitué par éponge, mousse et coton.

3. Dispositif (100) selon la revendication 1, dans lequel l'extrémité avant (106-1) est un couvercle de gaine fixé de manière amovible avec l'ensemble gaine (106).

4. Dispositif (100) selon la revendication 1, dans lequel l'extrémité avant (106-1) est revêtue d'un anticoagulant.

5. Dispositif (100) selon la revendication 1, dans lequel l'ensemble gaine (106) est configuré pour être inséré dans l'une quelconque d'une ligne artérielle (302-1) et d'une ligne veineuse (302-2) du cathéter (302) pour la désinfection.

6. Procédé non thérapeutique pour la désinfection d'un cathéter (302), le procédé comprenant :
l'insertion d'un dispositif de désinfection (100) pour la désinfection du cathéter (302) dans le cathéter (302), le dispositif (100) comprenant :
un élément déployable (104) fixé au niveau d'une partie avant d'une tige (102) et humidifié avec un désinfectant ; et
un ensemble gaine (106) logeant la tige (102) et l'élément déployable (104), l'ensemble gaine (106) comportant une extrémité avant (106-1) qui est fermée et une pluralité de fentes (108) qui sont présentes près de l'extrémité avant (106-1) ;
la poussée de l'élément déployable (104) vers l'extrémité avant (106-1) en actionnant la tige (102), pour permettre le déploiement vers l'extérieur de l'élément déployable (104) à travers la pluralité de fentes (108) lorsque l'élément déployable (104) atteint l'extrémité avant (106-1), l'élément déployable (104) mouillé avec le désinfectant et présent sous une forme déployée vers l'extérieur entrant en contact avec une surface interne du cathéter (302) ; et
la traction du dispositif (100) hors du cathéter (302) lorsque l'élément déployable (104) est présent dans la forme déployée vers l'extérieur, désinfectant de ce fait le cathéter (302).

7. Procédé non thérapeutique selon la revendication 6, comprenant la rotation du dispositif (100) tout en tirant le dispositif (100) hors du cathéter (302).

8. Procédé non thérapeutique (500) selon la revendication 6, dans lequel l'insertion du dispositif (100) dans le cathéter (302) comprend l'insertion de l'ensemble gaine (106) dans l'une quelconque d'une ligne artérielle (302-1) et d'une ligne veineuse (302-2) du cathéter (302).

9. Procédé non thérapeutique (500) selon la revendication 8, comprenant :
le rinçage de la ligne artérielle (302-1) et de la ligne veineuse (302-2) du cathéter (302) avec une solution saline avant d'insérer le dispositif (100) à l'intérieur ; et
l'élimination de la solution saline résiduelle de la ligne artérielle (302-1) et de la ligne veineuse (302-2) après avoir tiré le dispositif (100) hors du cathéter (302).

10. Procédé non thérapeutique (500) selon la revendication 6, dans lequel l'élément déployable (104) est tiré vers l'arrière à l'intérieur de l'ensemble gaine (106) après achèvement d'une procédure de désinfection, pour une mise au rebut sûre du dispositif (100).
